# EUROPÄISCHE PATENTSCHRIFT

(11) **EP 0 918 423 B1**
(45) Veröffentlichungstag und Bekanntmachung des Hinweises auf die Patenterteilung: **10.03.2004**
(21) Anmeldenummer: 98118755.2
(22) Anmeldetag: 05.10.1998
(51) Int. Cl.: H04L 29/06

(54) **Mobiles Telefon für Internet-Anwendungen**
Mobile phone for Internet applications
Téléphone mobile pour applications Internet

(30) Priorität: 15.10.1997 DE 19745539
(43) Veröffentlichungstag der Anmeldung: 26.05.1999
(73) Patentinhaber: Nokia Corporation, 02150 Espoo (FI)
(72) Erfinder: Theimer, Wolfgang, 44795 Bochum (DE)
(74) Vertreter: TER MEER STEINMEISTER & PARTNER GbR

(56) Entgegenhaltungen:
- WO-A-97/26750
- KENNEDY P: "MobileWeb/sup TM/ changing the face of mobile networking through universal wireless connectivity" IEEE TECHNICAL APPLICATIONS CONFERENCE. NORTHCON/96. CONFERENCE RECORD (CAT. NO.96CH35928), IEEE TECHNICAL APPLICATIONS CONFERENCE. NORTHCON/96. CONFERENCE RECORD, SEATTLE, WA, USA, 4-6 NOV. 1996, Seiten 89-94, XP002128907 1996, New York, NY, USA, IEEE, USA ISBN: 0-7803-3277-6
- "Nokia 9000 Technical Specifications" , [Online] 21. Juni 1997 (1997-06-21), Seiten 1-2, XP002128908 Retrieved from the Internet: <URL:http://www.midnet.ie/cellworld/specif i.html> [retrieved on 2000-01-27]

## Beschreibung

Die Erfindung betrifft ein mobiles Telefon gemäß dem Oberbegriff des Patentanspruchs 1, das insbesondere in einem Kommunikationssystem zur Überwachung und/oder Führung eines Fahrzeugs oder zur medizinischen Überwachung eines Patienten verwendet werden kann.

Kommunikationssysteme zum Beispiel zur Überwachung und/oder Führungs eines Fahrzeugs sind allgemein bekannt. So gibt es etwa bereits Fahrzeuge (Internet-Autos), die an das Internet angekoppelt sind, was einem Fahrer des Fahrzeugs eine Reihe technisch interessanter Möglichkeiten eröffnet. Nachteil derartiger Fahrzeuge ist allerdings, daß im Innern des Fahrzeugs ein als eigenständiger Web-Server programmierter internetfähiger Fahrzeugcomputer vorhanden sein muß, der über ein schnurloses mobiles Telefon mit dem Internet verbunden ist. Derartige internetfähige Fahrzeugcomputer brauchen jedoch zum einen sehr viel Platz und sind zum anderen relativ teuer.

Ein gattungsgemäßes mobiles Telefon, das nur einen Web-Browser enthält, ist aus "Nokia 9000 Technical Specifications", [Online] 21. Juni 1997 (1997-06-21), Seiten 1 - 2, XP002128908 Retrieved from the Internet: <URL: http: / / www.midnet.ie/cellworld/specif i.html> bekannt.

Work stations for client-server-Anwendungen sind bereits bekannt durch: Petri Pöyhönen "GSM PLMN makes a mobile office viable" Nokia Cellular Systems Speakers' Papers. 6th World Telecommunications Forum, Part 2. Technical Symposium. Integration, Interoperation and Interconnection: The Way To Global Services, Geneva, Switzerland 10-15 Oct. 1991 page 375-378. Vol 2. XP-0010332560. Eine derartige aus PC und Mobiltelefon aufgebaute Workstation dient als Datenbasis-Endgerät zur Eingabe von Anfragen sowie zum Empfang von Antworten von einem Datenbasis-Server, den sich die Workstation mit anderen Workstations teilt. Die Workstation hat hier also Browser-Funktion.

Der Artikel von B.N. Schilit et al., "TeleWeb: Lossely connected access to the World Wide Web", Computer Networks and ISDN Systems 28 (1996) Seiten 1431-1444, Elsvier Science B.V., befasst sich mit einem System, das jemandem ermöglicht jederzeit und überall im WEB zu browsen, egal ob er mit einem Netzwerk verbunden ist oder nicht.

Insbesondere befassen sich B.N. Schilit et al. mit dem verbesserten Internetzugang für ein Laptop, also für einen beweglichen Computer, und beschreiben in einem Scenario, wie mit Hilfe des TeleWeb ein Laptop überall dort mit dem World Wide Web verbindbar ist, wo ein wie auch immer gearteter Telefonanschluss vorhanden ist, also beispielsweise am Flughafen, im Flugzeug, im Hotel, usw.

Um die Möglichkeiten des Internetzugangs mittels eines Browsers im tragbaren Computer zu verbessern ist im tragbaren Computer ein sogenannter TeleWeb Daemon vorgesehen, der als "caching proxy" dient. Dieser caching proxy empfängt Anfragen von dem Browser im tragbaren Computer oder Laptop oder leitet diese entweder an das Netzwerk für einen Zugriff zum World Wide Web weiter oder, in dem Fall, dass die gewünschte Internetseite bereits im TeleWeb cach gespeichert ist, wird diese vom caching proxy selbst dem Browser zur Verfügung gestellt.

Aufgabe der Erfindung ist es, ein mobiles Telefon der eingangs genannten Art so weiterzubilden, daß mit ihm eine einfachere Kommunikation im Internet möglich ist.

Die Lösung der gestellten Aufgabe ist dem kennzeichnenden Teil des Patentanspruchs 1 zu entnehmen. Vorteilhafte Ausgestaltungen sind in den Unteransprüchen dargestellt.

Das mobile Telefon enthält mindestens einen WEB-Server. Ein WEB-Server ist dabei ein Software-Paket, das bestimmte Informationen über eine Schnittstelle zum Internet zur Verfügung stellt, die auf Anforderung von anderen, mit dem Internet verbundenen Einrichtungen abgefragt werden kann. Dadurch daß der WEB-Server im mobilen Telefon enthalten ist, wird auf einfache Weise ein lokal unabhängiger WEB-Server gebildet, der sich zu jeder Zeit beim Benutzer des mobilen Telefons befindet, falls dieser das Telefon mit sich führt.

Alternativ kann der WEB-Server die Information auch über eine Schnittstelle an ein lokales Netzwerk (LAN) oder ein anderes Netzwerk liefern. Desweiteren ist es denkbar, daß mehrere WEB-Server in einem mobilen Telefon enthalten sind, wobei zum Beispiel ein WEB-Server mit dem Internet verbunden ist, während ein anderer WEB-Server etwa mit einem lokalen Netzwerk verbunden sein kann. Dabei ist es auch denkbar, daß die einzelnen im mobilen Telefon enthaltenen WEB-Server untereinander gekoppelt sind.

Der Erfindung nach ist der mindestens eine WEB-Server in der Mikroprogrammsteuereinheit (MCU) des mobilen Telefons enthalten. Durch die Implementierung des WEB-Servers in der in einem mobilen Telefon bereits vorhandenen Mikroprogrammsteuereinheit braucht das mobile Telefon nicht mit zusätzlichen Komponenten erweitert zu werden.

Nach einer bevorzugten Weiterbildung der Erfindung ist der mindestens eine WEB-Server mit mindestens einem weiteren Server koppelbar. Dadurch kann auf jedem Server inhaltlich zusammengehörige Information enthalten sein, was einen schnellen Zugriff auf die entsprechende Information, zum Beispiel zur Aktualisierung, wesentlich vereinfacht. Dabei braucht der weitere Server lediglich bei Bedarf mit dem WEB-Server gekoppelt zu sein, wobei alternativ auch eine ständige Verbindung denkbar ist.

Nach einer weiteren Ausgestaltung der Erfindung ist der weitere Server im mobilen Telefon enthalten, wodurch er ebenfalls lokal unabhängig wird. Es kann sich bei dem weiteren Server aber auch um einen außerhalb des mobilen Telefons angeordneten handeln.

Ferner ist es möglich, daß auch mehrere Server im mobilen Telefon enthalten sind oder mehrere externe Server über eine Luftschnittstelle mit dem mobilen Telefon verbunden sind. Falls mehrere Server vorhanden sind, können diese zum Beispiel untereinander ständig oder bei Bedarf miteinander gekoppelt sein. Mit Hilfe eines externen Servers kann zum Beispiel spezielle Information von einem Service-Provider über eine Luftschnittstelle an den im mobilen Telefon enthaltenen WEB-Server übertragen werden. Dies ist dann von Vorteil, wenn die Information so umfangreich ist, daß ein Speichern dieser Information auf dem lokal unabhängigen WEB-Server aus Platzgründen nicht möglich ist.

Der Unterschied zwischen dem im mobilen Telefon enthaltenen WEB-Server und den im mobilen Telefon enthaltenen Servern liegt darin, daß lediglich der WEB-Server zum Beispiel über eine Luftschnittstelle mit einem Netzwerk (Internet, LAN) koppelbar ist.

Nach einer anderen Ausgestaltung der Erfindung ist der im mobilen Telefon enthaltene WEB-Server mit mindestens einem weiteren Client koppelbar. Ein Client ist ein Software-Paket, das von einem Server, also einem zweiten Software-Paket, Information erfragt. Dabei ist der mindestens eine weitere Client im mobilen Telefon enthalten.

Denkbar ist auch, daß der im mobilen Telefon enthaltene WEB-Server mit einem externen Client koppelbar ist, der zum Beispiel als autorisierter Browser ausgebildet ist und über die Luftschnittstelle Information vom WEB-Server abrufen kann.

Nach einer bevorzugten Ausführungsform der Erfindung ist der im mobilen Telefon enthaltene WEB-Server selbst als Client betreibbar. Dadurch kann der WEB-Server zum Beispiel von einem externen Server (Service-Provider) Information abrufen, wobei für den Zugriff auf den Service-Provider zum Beispiel der als WEB-Browser ausgebildete und im mobilen Telefon enthaltene Client verwendet werden kann. Ferner wird der WEB-Server als Client betrieben, wenn er Information von einem oder mehreren Servern, die im mobilen Telefon enthalten oder aber extern ausgebildet sein können, abruft.

Gemäß einer vorteilhaften Weiterbildung des erfindungsgemäßen mobilen Telefons wird dieses in einem Kommunikationssystem zur Überwachung und/oder Führung eines Fahrzeugs verwendet. Somit kann aufrelativ einfache Weise eine Kontrolle und Steuerung des Fahrzeugs durch den Fahrer oder eine externe Leitstelle erfolgen.

Nach einer anderen bevorzugten Ausführungsform des erfindungsgemäßen mobilen Telefons wird dieses in einem Kommunikationssystem zur medizinischen Überwachung eines Patienten eingesetzt. Dadurch ist es möglich, medizinische Daten zur Kontrolle des Gesundheitszustands von einer zentralen Leitstelle oder vom Hausarzt aus zu erfragen und eventuell notwendige Aktionen einzuleiten. Alternativ kann der Benutzer des mobilen Telefons mittels des im Telefon befindlichen WEB-Browsers die für seinen Gesundheitszustand relevante Information abrufen.

Die Erfindung wird im folgenden unter Bezugnahme auf die beigefügten Zeichnungen näher beschrieben. Es zeigen:
**Figur 1** ein Blockdiagramm eines Kommunikationssystems zur Überwachung eines Patienten, in dem das erfindungsgemäße mobile Telefon verwendet wird;
**Figur 2** ein Blockdiagramm der Implementierung des Kommunikationssystems nach Figur 1;
**Figur 3** ein Blockdiagramm eines Kommunikationssystems zur Überwachung und/oder Führung eines Fahrzeugs, in dem das erfindungsgemäße mobile Telefon verwendet wird; und
**Figur 4** ein Blockdiagramm der Implementierung des Kommunikationssystem nach Figur 3.

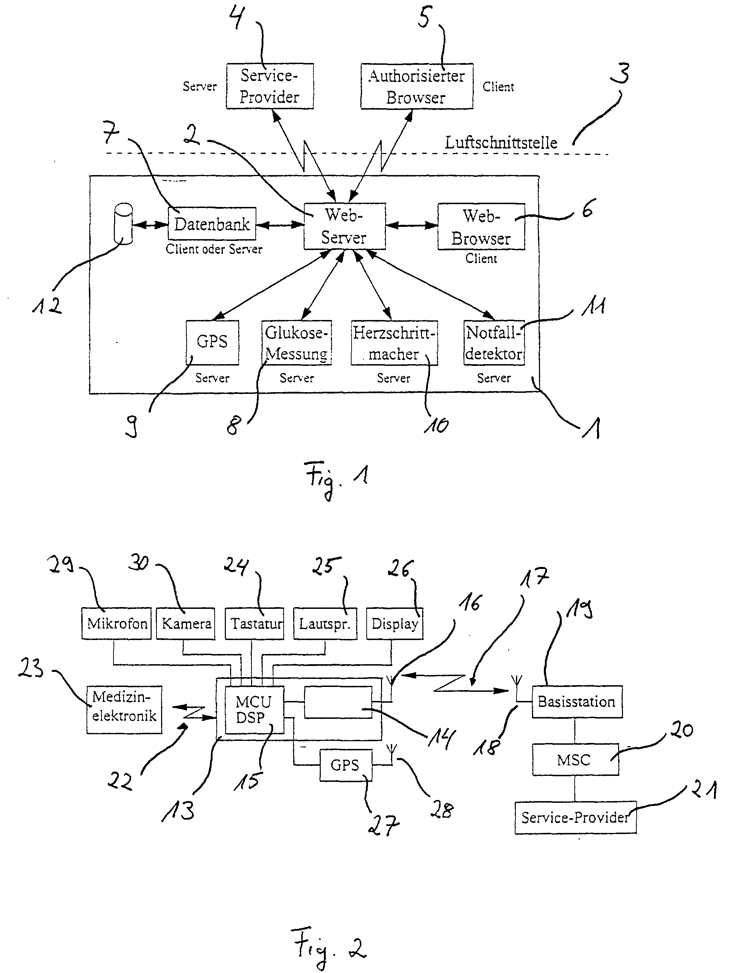

Figur 1 zeigt ein Blockdiagramm eines Kommunikationssystems, bei dem das erfindungsgemäße mobile Telefon (nicht gezeigt) verwendet wird, um einen Patienten medizinisch zu überwachen, wobei der in Figur 1 gezeigte Block 1 im mobilen Telefon enthalten ist.

Das mobile Telefon enthält folglich einen WEB-Server 2, der über eine Luftschnittstelle 3 mit einem als Server dienenden Service-Provider 4 koppelbar ist. Desweiteren ist der WEB-Server 2 über die Luftschnittstelle 3 mit einem als Client ausgebildeten autorisierten Browser 5 koppelbar. Der externe Service-Provider 4 und der externe autorisierte Browser 5 sind demnach über das Funknetz mit dem mobilen Telefon gekoppelt, wobei die Datenkommunikation über den im mobilen Telefon enthaltenen WEB-Server 2 erfolgt, der entweder Anfragen eines mobilen Browsers 6 nach außen weitergibt oder Anfragen von außen entgegennimmt und auswertet. Zur Auswertung wird eine lokale Datenbank 7 hinzugezogen, die in diesem Fall als Server fungiert. Umgekehrt kann die Datenbank 7 auch Client sein, wobei zur Aktualisierung ihres Datenbestands angeschlossene Geräte (zum Beispiel ein Glukosemeßsensor) über den WEB-Browser 2 abgerufen werden. WEB-Browser 6 und lokale Datenbank 7 sind ebenfalls im mobilen Telefon vorhanden.

Die von dem Glukosemeßsensor gemessenen Daten werden an einen im mobilen Telefon enthaltenen Glukosemessungs-Server 8 übertragen und dort gespeichert. Somit kann zum Beispiel ein medizinischer Service-Computer (autorisierter Browser 5) periodisch über den WEB-Server 2 die medizinischen Meßwerte (hier Glukosekonzentration) abfragen und bei Notfällen Instruktionen schicken. Bei akuten Notfällen (zum Beispiel Unterzuckerung) kann aber auch über den mobilen WEB-Browser 6 automatisch oder manuell Hilfe angefordert werden. Zur gezielten Notfallführung kann ein Service-Rechner über den autorisierten Browser 5, der seine Zugangsberechtigung durch ein Passwort oder eine digitale Signatur nachweist, und den WEB-Server 2 von einem im mobilen Telefon enthaltenen GPS-Server 9 die Position des in Not geratenen Patienten erfragen.

Das mobile Telefon enthält ferner einen Herzschrittmacher-Server 10, der Information über den Arbeitsbereich des Herzschrittmachers enthält.

Desweiteren enthält das mobile Telefon einen Notfalldetektor-Server 11, der zum Beispiel über einen Beschleunigungssensor Information darüber erhält, ob der Patient gestürzt ist. Diese Information kann über den WEB-Server 2 jederzeit abgerufen werden, wobei in einem Notfall der WEB-Server 2 über den WEB-Browser 6 automatisch über die Luftschnittstelle 3 Hilfe anfordern kann.

Zur Auswertung der im GPS-Server 9, Glukosemessungs-Server 8, Herzschrittmacher-Server 10 und Notfalldetektor-Server 11 enthaltenen Information wird diese über den WEB-Server 2 an die Datenbank 7 übertragen, die mit einem weiteren Speichermedium 12 gekoppelt ist. Die Datenbank 7 ist folglich als Client oder Server betreibbar.

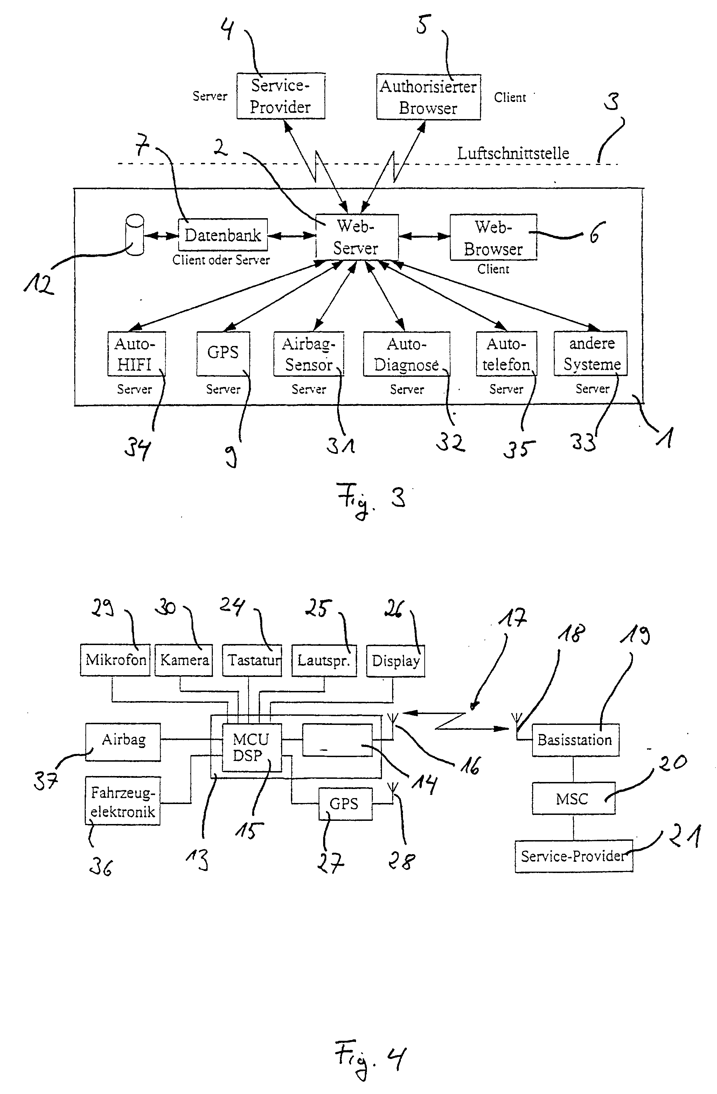

Figur 2 zeigt ein Blockdiagramm der Implementierung des medizinischen Kommunikationssystems nach Figur 1.

WEB-Server und -Browser sind Standardapplikationen, die für die konkreten Anwendungen lediglich etwas zugeschnitten werden müssen. Alle anderen Server können als C / C++-Programme realisiert werden, die Zugriff auf die Hardware besitzen (zum Beispiel Glukosemeßeinrichtung oder der GPS-Empfänger). Sie werden an den WEB-Server über ein CGI (common gateway interface) angeschlossen. Bei größeren Datenmengen ist es aufgrund der besseren Effizienz ratsam, die POST-Zugriffsmethode zu verwenden. Der Gateway-Server kommuniziert dabei mit dem WEB-Browser über Standard-Input und -Output.

Weil diese Teile des Systems nicht nach außen sichtbar sind, können sie leicht durch andere Technologien (zum Beispiel JAVA oder VRML) ausgetauscht werden. Als Ersatz für die im mobilen Einsatz nur beschränkt brauchbare Festplatte ist die Datenspeicherung in RAM oder FLASH vorgesehen.

Im Blockdiagramm nach Figur 2 ist ein mobiles Telefon 13 enthalten, in dem eine Sendeempfangseinheit 14 sowie eine Mikroprogrammsteuereinheit 15 (MCU) mit einem DSP vorhanden sind.

Der in Figur 1 gezeigte Block 1 ist bei dem Kommunikationssystem zur medizinischen Überwachung eines Patienten vollständig in der Mikroprogrammsteuereinheit 15 des erfindungsgemäßen mobilen Telefons 13 enthalten.

Über eine erste Antenne 16 ist das mobile Telefon 13 über die Sendeempfangseinheit 14 und eine Luftschnittstelle 17 mit einer eine Antenne 18 enthaltenden Basisstation 19 gekoppelt. Die Basisstation 19 kann zum Beispiel in einem GSM-System enthalten und über eine Mobilvermittlungsstelle (MSC) 20 mit einem Service-Provider 21 gekoppelt sein.

Das mobile Telefon 13 ist ferner über eine Schnittstelle 22 mit einer Medizinelektronik 23 gekoppelt. Diese Medizinelektronik 23 enthält einen Glukosemeßsensor zur Bestimmung der Glukosekonzentration einer Person mit Diabetes oder einer anderen Stoffwechselerkrankung, die den Zuckerhaushalt beeinflußt. Die Glukosekonzentration kann dabei automatisch periodisch gemessen werden, wobei die Resultate über die Schnittstelle 22 an das mobile Telefon 13 übertragen werden. Der Transfer erfolgt bevorzugt drahtlos (zum Beispiel über eine HF-Übertragung mit geringer Leistung und Reichweite), um keine permanente mechanische Verbindung zwischen Meßgerät und Mobiltelefon haben zu müssen. Das Meßgerät sollte Werte bei Unterbrechung der Datenübertragung zusammen mit ihrer Entstehungszeit puffern. Alternativ können anstelle der periodischen automatischen Messung durch eine entsprechende Sensorelektronik die Blutzuckerwerte auch regelmäßig durch die Person mit Diabetes über eine Tastatur 24 in das Mobilterminal eingegeben werden.

Die Medizinelektronik 23 enthält ferner einen Herzschrittmacher mit einem Funkempfangs- und Sendemodul. Sobald problematische Arbeitsbereiche des Herzschrittmachers detektiert werden (zum Beispiel dauerhafte Überlastung wegen zu hoher körperlicher Aktivität, technische Probleme des Geräts) wird eine Nachricht über die Schnittstelle 22 an das in der Nähe befindliche Mobiltelefon 13 gesendet und über den WEB-Server 2 aus Figur 1 in der lokalen Datenbank 7 gespeichert. Wie bereits oben beschrieben, kann bei der Detektion eines problematischen Arbeitsbereichs des Herzschrittmachers der in der MCU 15 des Mobiltelefons 13 enthaltene WEB-Browser 6 automatisch gestartet werden, wobei zum Beispiel über einen Lautsprecher 25 eine akustische oder über ein Display 26 eine optische Warnnachricht ausgegeben werden kann. Alternativ kann auch über die Schnittstelle 17, die Basisstation 19 und die MSC 20 eine Warnnachricht an den Service-Provider 21 gesendet werden.

Die Medizinelektronik enthält weiter eine automatische Hilfeanforderung, die zum Beispiel über einen Beschleunigungssensor auszulösen ist, der Stürze oder Unfälle detektiert. Eine weitere technisch einfache Lösung besteht darin, daß der Patient sich per WEB-Browser periodisch bei einer Service-Stelle meldet. Bleibt die Nachricht aus, wird eine Sprachverbindung zum Patienten zur Rückfrage aufgebaut. Antwortet der Patient nicht, löst das Service-Center aufgrund des möglichen Notfalls eine Hilfsaktion aus. Der externe autorisierte Browser 5 aus Figur 1 kann über den WEB-Server des Telefons nachfragen und evtl. von einem GPS-Empfänger 27 die genaue Position der Person erfragen. Der GPS-Empfänger 27 kann dabei ebenfalls im Mobiltelefon 13 integriert sein, wobei er über eine zweite Antenne 28 die GPS-Satellitensignale empfängt.

Das mobile Telefon 13 enthält zusätzlich ein Mikrophon 29 sowie eine Kamera 30. Mit Hilfe des Mikrophons kann ein zum Beispiel gestürzter Patient Hilfe anfordern, falls er nicht mehr in der Lage ist, die Tastatur 24 zu bedienen. Das Mikrophon 29 kann sich zum Beispiel automatisch aktivieren, wenn der o. g. Beschleunigungssensor eine Hilfeanforderung auslöst.

Mit Hilfe der Kamera 30 kann eine Ferndiagnose eines Patienten durchgeführt werden, wobei bei einer Notbehandlung per Mobilfunk ein behandelnder Arzt sich auch visuell einen Eindruck von dem Patienten verschaffen kann.

Figur 3 zeigt ein Blockdiagramm eines Kommunikationssystems zur Überwachung und/oder Führung eines Fahrzeugs, in dem das erfindungsgemäße mobile Telefon verwendet wird, wobei für gleiche Bestandteile die gleichen Bezugsziffern wie in Figur 1 und 2 verwendet werden.

Für Fahrzeuganwendungen läßt sich mit geringen Änderungen das gleiche System aus Figur 1 und 2 verwenden. Die Datenverbindung zu medizinischen Geräten muß dabei lediglich durch Interfaces zur Fahrzeugelektronik und anderen Einbaugeräten ersetzt werden.

Figur 3 zeigt einen in einem mobilen Telefon (zum Beispiel in der MCU des mobilen Telefons) enthaltenen Block 1 mit einem WEB-Server 2, der mit einem als Client ausgebildeten WEB-Browser 6 gekoppelt ist.

Das in Figur 3 gezeigte Kommunikationssystem kann zum Beispiel zur Fahrzeugnavigation verwendet werden. Dazu fordert ein Benutzer über den WEB-Browser 6 von einem Service-Provider 4 eine Routenplanung an, indem er das Ziel und die Randbedingungen eingibt. Die Anforderung wird dann in eine Warteschlange eines Datenbank-Servers 7 eingeordnet. Der Datenbank-Client 7 verarbeitet nun die Anforderungen dadurch, daß er von einem GPS-Server 9 die aktuelle Position und von einem Airbag-Server 31 sowie von einem Diagnoseserver 32 den aktuellen Sicherheitszustand erfragt. Diese Information wird dann zum Service-Provider 4 geschickt. Als Antwort erhält der Fahrer eine lokale Straßenkarte zum Ziel, auf der der optimale Weg markiert ist. Der WEB-Browser 6 fragt nun periodisch den Datenbank-Server 7 ab, um für die aktuelle Position visuelle Informationen auf einem Bildschirm (in Figur 3 nicht gezeigt) auszugeben bzw. den Fahrer durch situationsgerechte akustische Meldungen zu leiten. Der Datenbasis-Client 7 bleibt im Hintergrund aktiv und verfolgt die GPS-Position des Fahrzeugs. Erreicht die Fahrzeugposition die Grenzen der lokalen Karte, sendet der Client automatisch eine Anfrage an den Service-Provider 4, um die lokale Karte zu aktualisieren. Zwischen zwei solchen Anfragen ist keine externe Datenverbindung nötig, da alles Wissen für die lokale Routenplanung sich im Fahrzeug befindet.

Der Datenbank-Client 7 überwacht den Sicherheitszustand des Fahrzeugs dadurch, daß er periodisch Anfragen an den GPS 9-,den Airbag 31- und den Diagnose-Server 32 sendet, um kritische Situation zu detektieren. Falls ein Unfall oder andere gravierende Störungen auftreten, sendet der Datenbank-Client 7 automatisch einen Notruf an den Service-Provider. Dieser antwortet mit einer Beschreibung der zugehörigen von ihm einzuleitenden Aktion und öffnet einen Sprachkanal zum Fahrer. Damit kann ggf. der Gesundheitszustand der Fahrzeuginsassen nachgefragt werden oder eine Hilfsaktion effektiv geplant werden.

Figur 3 zeigt ferner einen als Client ausgebildeten autorisierten Browser 5, über den eine zentrale, beim Spediteur ausgebildete Transport-Datenbank (nicht gezeigt) auf die gesamte Fahrzeugflotte Zugriff besitzt. Ist eine Flottenmanagement-Applikation im Fahrzeug aktiv, sendet der lokale Datenbank-Client 7 periodisch GPS-Position, Ziel, Zustand und Fracht des Fahrzeugs zum zentralen Transport-Datenbank-Server, der diese Information speichert.

Das in Figur 3 gezeigte Kommunikationssystem kann ferner zum Diebstahlschutz und zur Fahrzeugverfolgung verwendet werden, wobei der Diebstahlschutz vom Fahrzeugbesitzer durch Senden eines Autorisierungsschlüssels an den Service-Provider 4 initiiert werden sollte. Falls jemand unerlaubt in das Fahrzeug eindringt, wird der lokale Datenbank-Client 7 automatisch gestartet, fragt periodisch vom GPS-Server 9 die aktuelle Position ab und sendet eine Alarmnachricht mit der Position zum Service-Provider 4. Dieser kann nun das Fahrzeug durch Eingriff in die Motorelektronik stillegen. Alternativ kann die Position an die Polizeifahndung übermittelt werden.

Die Frage nach dem technischen Zustand eines Fahrzeugs bzw. dessen Instandhaltung kann durch Abfrage des Autodiagnose-Servers 32, der Zugriff auf die relevanten technischen Systeme im Auto hat, beantwortet werden. Die Auswertung der Diagnosedaten könnte nach Abfrage der Information mittels des WEB-Browsers 6 in einem lokal angeschlossenen Service-Computer erfolgen. Andernfalls kann diese Information von dem autorisierten externen WEB-Browser 5 abgefragt werden und in einer Service-Stelle (zum Beispiel Kfz-Werkstatt) ausgewertet werden. Mit dem letzteren Vorgehen kann ein entfernter Experte die Auswertung der Fahrzeugfehler durchführen.

Wie in Figur 3 gezeigt, enthält der Block 1 ferner einen Server 33 für andere Systeme. Dieser Server kann zum Beispiel zur Kontrolle von Fahrzeuggeräten, wie etwa der Heizung, verwendet werden. Die Kontrolle erfolgt dabei über den lokalen WEB-Browser 6 oder aber über den externen autorisierten Browser 5. Auf diese Weise ist es möglich, schon zu Hause oder am Arbeitsplatz die Fahrzeugheizung zu aktivieren.

Der WEB-Server 2 ist ferner mit einem Auto-HIFI-Server gekoppelt, auf dem zum Beispiel komprimierte Audio-Information abgespeichert ist. In Mobilfunkgeräten der dritten Generation (UMTS, Nachfolger von GSM) kann eine erheblich höhere Datenmenge übertragen werden. Damit wird es möglich sein, personenzugeschnittene Internet-Radio- und Videodienste anzubieten, sofern die Gebühren dafür attraktiv sind und eine einfache automatische Abbuchung existiert. Ein Benutzer tuned sich auf einen Internet-Kanal mittels Push-Technologie (d. h. er erhält ohne Nachfrage die aktuelle Information des Kanals, zum Beispiel ein Radioprogramm). Alternativ kann ein Benutzer seinen privaten WEB-Server kontaktieren, um sein gewünschtes Programm zu transferieren (zum Beispiel eine komprimierte Audio-CD). Die empfangenen Daten können komprimierte Audio-Information, MIDI-Musik oder komprimierte Video-Information sein. Diese Information kann vom Benutzer über den WEB-Browser 6 und den WEB-Server 2 abgerufen werden, wodurch das mobile Internet-Terminal ein Radio und einen tragbaren Fernseher ersetzt.

Figur 3 zeigt weiter einen im Block 1 enthaltenen Auto-Telefon-Server 35, der mit dem WEB-Server 2 gekoppelt ist, und auf den über den WEB-Browser 6 oder aber über den autorisierten Browser 5 zugegriffen werden kann.

Figur 4 zeigt ein Blockdiagramm der Implementierung des Kommunikationssystems nach Figur 3, wobei für gleiche Komponenten die gleichen Bezugsziffern wie in Figur 2 verwendet werden.

Figur 2 und Figur 4 unterscheiden sich lediglich dadurch, daß das mobile Telefon 13 in Figur 4 statt mit einer Medizinelektronik mit einer Fahrzeugelektronik 36 sowie einem Airbag-Sensor 37 gekoppelt ist (fest oder über Luftschnittstelle).

Der Airbag-Sensor 37 liefert kontinuierlich Signale an den Airbag-Sensor-Server 31 aus Figur 3, wobei bei einem Unfall der Datenbank-Client 7 aus Figur 3, der kontinuierlich den Airbag-Sensor-Server 31 abfragt, automatisch einen Notruf an den Service-Provider 21 über die Luftschnittstelle 17 sendet.

Die Fahrzeugelektronik 36 sendet ebenfalls kontinuierlich Daten an den Autodiagnose-Server 32 aus Figur 3. Wie bereits oben beschrieben, kann auf diesen Autodiagnose-Server 32 bei Bedarf zugegriffen werden, wobei auch über den autorisierten Browser 5 in die Fahrzeugelektronik eingegriffen werden kann.

## Patentansprüche

1. Mobiles Telefon mit einem WEB-Browser, **dadurch gekennzeichnet, dass** mindestens ein WEB-Server, der Anfragen von außen entgegennimmt und auswertet, in der Mikroprogrammsteuereinheit (MCU) des mobilen Telefons enthalten ist.

2. Mobiles Telefon nach Anspruch 1, **dadurch gekennzeichnet, daß** der mindestens eine WEB-Server mit mindestens einem weiteren Server koppelbar ist.

3. Mobiles Telefon nach Anspruch 2, **dadurch gekennzeichnet, daß** der weitere Server im mobilen Telefon enthalten ist.

4. Mobiles Telefon nach einem der Ansprüche 1 bis 3, **dadurch gekennzeichnet, daß** der WEB-Server mit mindestens einem weiteren Client (7) koppelbar ist der im mobilen Telefon enthalten ist.

5. Mobiles Telefon nach einem der Ansprüche 1 bis 4, **dadurch gekennzeichnet, daß** der WEB-Server selbst als Client betreibbar ist.

6. Verwendung des mobilen Telefons nach einem der Ansprüche 1 bis 5 in einem Kommunikationssystem zur Überwachung und / oder Führung eines Fahrzeugs.

7. Verwendung des mobilen Telefons nach einem der Ansprüche 1 bis 5 in einem Kommunikationssystem zur medizinischen Überwachung eines Patienten.

## Claims

1. Mobile telephone having a WEB browser **characterized in that** at least one WEB server, which receives and evaluates enquiries from the outside, is contained in the microprogram control unit (MCU) of the mobile telephone.

2. Mobile telephone according to Claim 1, **characterized in that** the at least one WEB server can be coupled to at least one further server.

3. Mobile telephone according to Claim 2, **characterized in that** the further server is contained in the mobile telephone.

4. Mobile telephone according to one of Claims 1 to 3, **characterized in that** the WEB server can be coupled to at least one further client which is contained in the mobile telephone.

5. Mobile telephone according to one of Claims 1 to 4, **characterized in that** the WEB server can itself be operated as a client.

6. Use of the mobile telephone according to one of Claims 1 to 5, in a communications system for monitoring and/or guiding a vehicle.

7. Use of the mobile telephone according to one of Claims 1 to 5, in a communications system for medically monitoring a patient.

## Revendications

1. Téléphone mobile, muni d'un navigateur WEB, **caractérisé en ce qu'**au moins un serveur WEB, qui reçoit et évalue depuis l'extérieur des requêtes, est contenu dans l'unité de commande à microprogramme (MCU) du téléphone mobile.

2. Téléphone mobile selon la revendication 1, **caractérisé en ce que** le au moins serveur WEB est susceptible d'être accouplé à au moins un autre serveur.

3. Téléphone mobile selon la revendication 2, **caractérisé en ce que** l'autre serveur est contenu dans le téléphone mobile.

4. Téléphone mobile selon l'une des revendications 1 à 3, **caractérisé en ce que** le serveur WEB est susceptible d'être susceptible d'être couplé à au moins un autre client (7), contenu dans le téléphone mobile.

5. Téléphone mobile selon l'une des revendications 1 à 4, **caractérisé en ce que** le serveur WEB lui-même peut fonctionner en tant que client.

6. Utilisation du téléphone mobile selon l'une des revendications 1 à 5, dans un système de communication pour la surveillance et/ou le guidage d'un véhicule.

7. Utilisation des téléphones mobiles selon l'une des revendications 1 à 5, dans un système de communication, servant à la surveillance médicale d'un patient.
